# EUROPEAN PATENT SPECIFICATION

(11) **EP 1 423 159 B1**
(45) Date of publication and mention of the grant of the patent: **09.05.2007**
(21) Application number: 02734329.2
(22) Date of filing: 09.05.2002
(51) Int. Cl.: A61M 25/00

(54) **ECCENTRIC CATHETER SHAFT**
EXZENTRISCHER KATHETERSCHAFT
TIGE DE CATHETER DECALEE

(30) Priority: 07.09.2001 US 948517
(43) Date of publication of application: 02.06.2004
(73) Proprietor: Boston Scientific Limited, St. Michael (BB)
(72) Inventor: HACKETT, Steven, S., Maple Grove, MN 55369 (US)
(74) Representative: Vossius & Partner
(86) International application number: PCT/US2002/014747
(87) International publication number: WO 2003/037415

(56) References cited:
- BE-A- 689 333
- US-A- 4 601 713
- US-A- 5 968 009

## Description

The present invention generally relates to intravascular medical devices. More specifically, the present invention relates to multi-lumen intravascular medical devices such as balloon catheters.

### Background of the Invention

Intravascular devices are commonly used to diagnose and treat various types of vascular disease. For example, coronary artery disease (CAD) may be treated utilizing a procedure called percutaneous transluminal coronary angioplasty (PTCA). In a typical PTCA procedure, intravascular devices are inserted into the patient's vascular system at a remote access site such as the femoral artery near the groin. The intravascular devices are navigated through the femoral artery and the descending aorta, over the aortic arch, down the ascending aorta, and into the targeted coronary artery.

The path from the remote access site to the targeted coronary artery is established and maintained utilizing a conventional guide catheter and guidewire. The guide catheter extends from a point outside the patient's body, through the remote access site, to the ostium of the targeted coronary artery. The guidewire extends from a point outside the patient's body, through the guide catheter, and across the treatment site of the targeted coronary artery. A balloon catheter may then be advanced over the guidewire through the guide catheter until the distally mounted balloon is positioned across the treatment site. The balloon is then inflated to dilate the vascular restriction, thereby opening the artery and restoring blood flow.

Different types of balloon catheters are suitable for use in this type of procedure. Balloon catheters that are designed for use in combination with a guidewire as discussed above are typically referred to as over-the wire (OTW) or rapid exchange (RX) type balloon catheters. OTW and RX type balloon catheters include an elongate shaft having an inflation lumen and a guidewire lumen. In an OTW type balloon catheter, the guidewire lumen extends from the proximal end of the catheter to the distal end of the catheter. In an RX type balloon catheter, the guidewire lumen extends from a point distal of the proximal end to the distal end of the catheter. In both cases, at least a portion of the elongate shaft includes an inflation lumen and a guidewire lumen.

In typical OTW and RX type balloon catheters, the guidewire lumen and the inflation lumen are defined by either a coaxial shaft structure or a dual lumen shaft structure. In a coaxial design, the elongate shaft includes an inner tube coaxially disposed in an outer tube such that the inner tube defines a circular guidewire lumen and the outer tube defines an annular inflation lumen. An example of a typical coaxial shaft design is disclosed in U.S. Patent No. 4,323,071 to Simpson et al. In dual lumen shaft designs, a single tubular extrusion is used to define separate guidewire and inflation lumens extending side-by-side. An example of a dual lumen shaft design is disclosed in U.S. Patent No. 4,782,834 to Maguire et al.

One advantage provided by a coaxial type shaft design, as compared to a dual lumen type shaft design, is uniform flexibility due to the coaxial arrangement of parts. In other words, the coaxial type shaft design has the same flexibility in all planes of flexure, whereas the dual lumen type shaft design has non-uniform flexibility in different planes of flexure due to the imbalance of material relative to the longitudinal center axis of the catheter shaft. The non-uniformity in flexibility of the dual lumen type shaft design may compromise trackability and torqueability of the catheter, thereby reducing the ability of the catheter to navigate tortuous vasculature. One advantage provided by a dual lumen type shaft design is reduced frictional loss and less resistance to fluid flow in the inflation lumen as compared to a coaxial type shaft design having the same cross-sectional area. This provides better balloon inflation/deflation rates which are desirable for various clinical reasons.

U.S. Patent No. 4,601,713 which describes the closest prior art, relates to a variable diameter catheter adapted to be longitudinally folded for insertion into a first end of a body orifice and then unfolded after insertion for subsequently transporting a fluid. The catheter comprises a resiliently flexible tube of generally uniform diameter along its length, said tube having a wall of varying thickness around its circumference, such that said wall is longitudinally foldable upon itself, with that portion of said wall which folds upon itself being thinner than the rest of the wall, and means for retaining a fold in said tube. The catheter tube has sufficient resilient memory, such that said folded tube will unfold to its normal rounded configuration upon removal or release of said retaining means.

Accordingly, there is a need for a shaft design for an intravascular device such as a balloon catheter wherein the flexibility is uniform in all planes of flexure and the frictional loss in the inflation lumen is minimized.

### Summary of the Invention

To address this need, the present invention provides an intravascular device, such as a balloon catheter, having a tubular shaft with an outer wall and an inner wall. The inner wall divides the outer wall into two or more lumens, such as a larger crescent-shaped lumen which may be used as an inflation lumen, and a smaller circle-shaped lumen which may be used as a guidewire lumen. The shaft also includes one or more regions of modified flexibility extending longitudinally along the outer wall. Absent the regions of modified flexibility, the inner wall would create an imbalance of material and flexibility about the center axis of the shaft. The regions of modified flexibility are positioned to reduce any such imbalance, thereby providing more uniform flexibility, without compromising the fluid dynamic capabilities of the lumens. The regions of modified flexibility also provide for more uniform torque transmission, and thereby reduce whipping effects.

In one embodiment, the regions of modified flexibility comprise one or more regions of decreased wall thickness in the outer wall. In another embodiment, the regions of modified flexibility comprise one or more spines extending longitudinally along the outer wall. In yet another embodiment, the regions of modified flexibility comprise a combination of these features.

### Brief Description of the Drawings

Figure 1 is a plan view of an intravascular device in accordance with the present invention, shown in the exemplary form of a balloon catheter;
Figure 2A is a cross-sectional view and Figure 3A is a partial isometric view of an embodiment of the elongate shaft of the intravascular device shown in Figure 1;
Figure 2B is a cross-sectional view and Figure 3B is a partial isometric view of another embodiment of the elongate shaft of the intravascular device shown in Figure 1;
Figure 2C is a cross-sectional view of a further embodiment of the elongate shaft of the intravascular device shown in Figure 1; and
Figure 2D is a cross-sectional view of yet another embodiment of the elongate shaft of the intravascular device shown in Figure 1.

### Detailed Description of the Invention

The following detailed description should be read with reference to the drawings in which similar elements in different drawings are numbered the same. The drawings, which are not necessarily to scale, depict illustrative embodiments and are not intended to limit the scope of the invention.

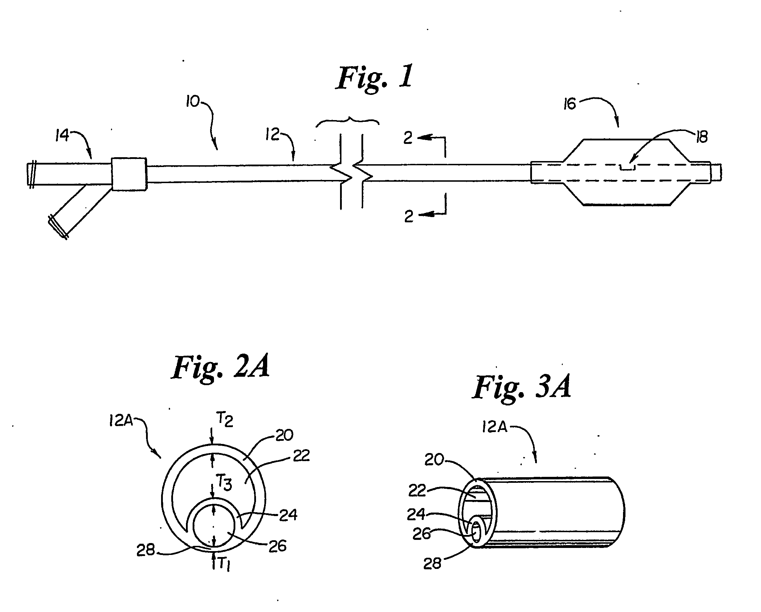

Refer now to Figure 1, which illustrates a plan view of an intravascular device in the form of a balloon catheter 10. Those skilled in the art will recognize that the present invention may be implemented in a wide variety of intravascular devices, such as infusion catheters, guide catheters, diagnostic catheters, atherectomy devices and balloon catheters such as balloon catheter 10. Balloon catheter 10 includes an elongate shaft 12 having a proximal end and a distal end. A conventional manifold 14 is connected to the proximal end of the elongate shaft 12. Manifold 14 facilitates connection to an inflation device to inflate and deflate a balloon 16 mounted to the distal end of the elongate shaft 12. Fluid communication between the manifold 14 and the inflatable balloon 16 is provided by way of an inflation lumen 22 (visible in Figure 2A) and an inflation port 18. Manifold 14 also facilitates insertion of a guidewire (not shown) into the guidewire lumen 26 (visible in Figure 2A) which extends to the distal end of the elongate shaft 12. With the exception of the elongate shaft 12 and its features discussed hereinafter, intravascular balloon catheter 10 is substantially conventional. Figures 2A-2D describe various embodiments (12A,12B,12C,12D) of the elongate shaft 12 of the intravascular balloon catheter 10 illustrated in Figure 1.

Refer now to Figure 2A, which illustrates a cross-sectional view of a first embodiment of the elongate shaft 12A taken along line 2-2 in Figure 1. Also refer to Figure 3A, which illustrates an isometric view of a segment of the elongate shaft 12A. In this particular embodiment, the elongate shaft 12A includes an outer wall 20 and an inner wall 24. As used herein for purposes of description, the outer wall 20 refers to the entire wall defining the circumference of the elongate shaft 12A, and the inner wall 24 refers to the wall segment extending between two points inside the outer wall 20.

The outer wall 20 defines the majority of the inflation lumen 22. The inner wall 24 and a portion of the outer wall 20 define the guidewire lumen 26. In this particular example, the inflation lumen 22 is crescent-shaped and larger than the circle-shaped guidewire lumen 26. Those skilled in the art will recognize that that size, shape and position of the inner wall 24 may be varied to change the size, shape and geometry of the inflation lumen 22 and the guidewire lumen 26. In addition, those skilled in the art will recognize that the lumens 22,26 may be varied in number and function depending on the particular intravascular device implementing the concepts of the present invention.

As seen in Figure 2A, the portion of the outer wall 20 which defines the guidewire lumen 26 includes a thinned portion 28 extending longitudinally along the shaft 12A. The thinned portion 28 of the outer wall 20 has a wall thickness T₁ which is less than the wall thickness T₂ of the remainder of the outer wall 20. The thickness T₁ of the thinned portion 28 may also be less than the wall thickness T₃ of the inner wall 24. The reduced wall thickness T₁ of the thinned portion 28 compensates for the imbalance of material and flexibility relative to the center longitudinal axis of the elongate shaft 12A due to the inner wall 24. In Figure 2A, the center longitudinal axis of the elongate shaft 12A appears as a point (not shown) positioned at the geometric center of the outer wall 20. The provision of the inner wall 24 increases the amount of material on one side of the shaft 12A when viewed in cross section. The increased amount of material due to the inner wall 24 increases the rigidity along that side of the elongate shaft 12A, thereby causing non-uniformity in flexibility in different planes of flexure. By reducing the wall thickness T₁ in the thinned outer wall portion 28, the imbalance of material and flexibility due to the inner wall 24 is mitigated.

Because the thinned portion 28 of the outer wall 20 does not define any portion of the inflation lumen 22, the thinned portion 28 does not compromise the ability of the inflation lumen 22 to withstand high inflation pressures. In addition, the inner wall 24 may be shifted toward the thinned portion 28 of the outer wall 20 a distance approximately equal to T₂-T₁ without compromising the size of the guidewire lumen 26. Because the inner wall 24 may be shifted in the direction of the thinned portion 28 of the outer wall 20, the inflation lumen 22 also benefits from a corresponding increase in cross-sectional area, thereby improving fluid flow therethrough.

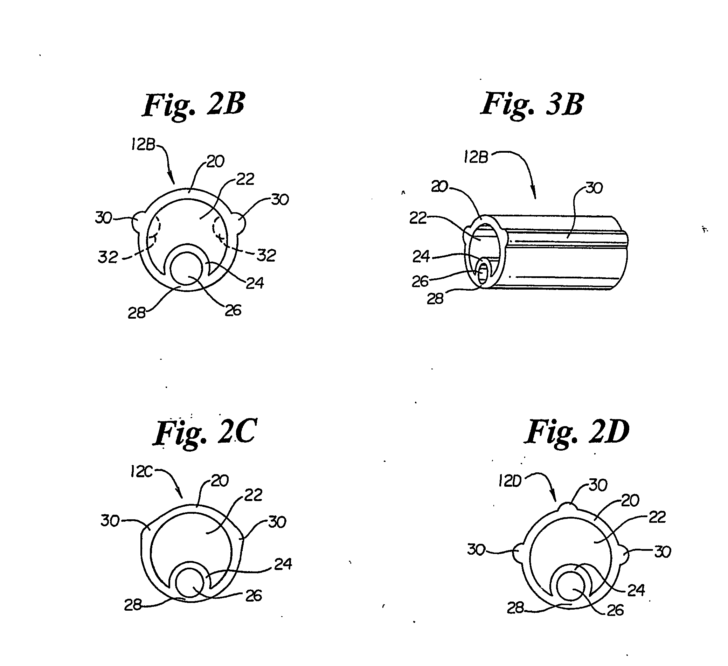

Refer now to Figure 2B, which illustrates a cross-sectional view of an elongate shaft 12B in accordance with another embodiment of the present invention. Also refer to Figure 3B, which illustrates an isometric view of a segment of the elongate shaft 12B. Except as illustrated and described herein, the elongate shaft 12B is substantially the same as elongate shaft 12A described with reference to Figures 2A and 3A.

Elongate shaft 12B includes an outer wall 20, an inner wall 24, an inflation lumen 22 and a guidewire lumen 26. Elongate shaft 12B may optionally include a thinned region 28 in the outer wall 20. Elongate shaft 12B further includes longitudinally extending spines 30 to further compensate for the imbalance of material and flexibility about the center longitudinal axis of the shaft 12B that would otherwise occur due to the inner wall 24. Relative to the center longitudinal axis, the longitudinal spines 30 are disposed on the opposite side of the inner wall 24 and the thinned portion 28 of the outer wall 20.

The longitudinal spines 30 may comprise discrete components connected to the outer wall 20. Alternatively, the longitudinal spines 30 may comprise integral components of the outer wall 20 such as an increase in thickness of the outer wall 20. Preferably, the longitudinal spines 30 are integrally formed with the outer wall 20 during extrusion. The longitudinal spines 30 may extend outwardly from the outer wall 20 (as shown) to maintain the size of the inflation lumen 22. Alternatively, the longitudinal spines 32 (shown in phantom) may extend inwardly into the inflation lumen 22 to maintain the outside profile of the elongate shaft 12B.

The longitudinal spines 30 may be positioned, relative to the center longitudinal axis of the elongate shaft 12B, opposite the inner wall 24 and the thinned portion 28 of the outer wall 20. The longitudinal spines 30 may be positioned equidistant from the inner wall 24 and/or thinned portion 28 of the outer wall 20. Preferably, the longitudinal spines 30 are uniformly spaced along the outer wall 20 opposite the inner wall 24 and thinned portion 28 of the outer wall 20 to increase the balance of material and flexibility about the center axis of the elongate shaft 12B.

Those skilled in the art will recognize that the size, shape and number of longitudinal spines 30 may be varied depending on the size, shape and position of the inner wall 24. For example, it is contemplated that a single longitudinal spine 30 may be positioned immediately opposite the inner wall 24 and thinned portion 28 of the outer wall 20. If two longitudinal spines 30 are utilized (as shown), the spines 30 may be positioned approximately one-third the radius of the outer wall 20 from the longitudinal center axis of the elongate shaft 12B to properly counterbalance the material of the inner wall 24.

As mentioned previously, the size, shape and number of longitudinal spines 30 may be varied depending on the degree of counterbalance needed to balance the material and flexibility of the elongate shaft 12. Figures 2C and 2D illustrate examples of variations in the size, number and position of the longitudinal spines 30. Figure 2C illustrates elongate shaft 12C having two longitudinal spines 30 with a smoother outside surface than elongate shaft 12B. Figure 2D illustrates an elongate shaft 12D having three longitudinal spines 30 uniformly spaced about the outer wall 20 opposite the inner wall 24 relative to the longitudinal center axis.

## Claims

1. An intravascular device comprising an elongate tubular shaft (12) having an outer wall (20) and an inner wall (24) dividing the outer wall (20) into first and second longitudinal lumens, and one or more regions of modified flexibility extending longitudinally along the outer wall to reduce an imbalance of material and flexibility about a longitudinal center axis of the shaft (12) that would otherwise occur absent the regions of modified flexibility.

2. An intravascular device as in claim 1, wherein the first lumen is larger than the second lumen.

3. An intravascular device as in claim 2, wherein the first lumen is crescent shaped.

4. An intravascular device as in claim 3, wherein the second lumen is circular.

5. An intravascular device as in claim 1, wherein the one or more regions of modified flexibility comprise one or more regions of decreased wall thickness in the outer wall.

6. An intravascular device as in claim 1, wherein the one or more regions of modified flexibility comprise one or more spines (30) extending longitudinally along the outer wall.

7. An intravascular device as in claim 6, wherein the spines (30) are integral with the outer wall (20).

8. An intravascular device as in claim 7, wherein the spines (30) comprise regions of increased wall thickness in the outer wall.

9. An intravascular device as in claim 8, wherein the spines (30) extend outwardly from the outer wall (20).

10. An intravascular device as in claim 8, wherein the spines (30) extend inwardly.

11. An intravascular device as in claim 1, wherein the one or more regions of modified flexibility comprise a combination of one or more regions of decreased wall thickness in the outer wall and one or more spines extending longitudinally along the outer wall.

12. An intravascular device as in claim 11, wherein the one or mole spines (30) are positioned, relative to the center longitudinal axis, opposite the inner wall (24).

13. An intravascular device as in claim 12, wherein the one or more spines (30) are positioned equidistant from the inner wall.

14. An intravascular device as in claim 12, wherein the one or more spines (30) are uniformly spaced.

## Patentansprüche

1. Intravaskuläre Vorrichtung mit einem längliche röhrenförmigen Schaft (12) mit einer Außenwand (20) und einer Innenwand (24), die die Außenwand (20) in ein erstes und zweites Längslumen teilt, und einem oder mehreren Bereichen modifizierter Flexibilität, die sich in Längsrichtung entlang der Außenwand erstrecken, um eine Unausgeglichenheit des Materials und der Flexibilität um eine Längsmittelachse des Schaftes (12) zu reduzieren, die andernfalls ohne die Bereiche modifizierter Flexibilität auftreten würde.

2. Intravaskuläre Vorrichtung nach Anspruch 1, wobei das erste Lumen größer ist als das zweite Lumen.

3. Intravaskuläre Vorrichtung nach Anspruch 2, wobei das erste Lumen sichelförmig ist.

4. Intravaskuläre Vorrichtung nach Anspruch 3, wobei das zweite Lumen kreisförmig ist.

5. Intravaskuläre Vorrichtung nach Anspruch 1, wobei der eine oder die mehreren Bereiche modifizierter Flexibilität einen oder mehrere Bereiche verringerter Wanddicke in der Außenwand aufweisen.

6. Intravaskuläre Vorrichtung nach Anspruch 1, wobei der eine oder die mehreren Bereiche modifizierter Flexibilität einen oder mehrere Rippen (30) aufweisen, die sich in Längsrichtung entlang der Außenwand erstrecken.

7. Intravaskuläre Vorrichtung nach Anspruch 6, wobei die Rippen (30) mit der Außenwand (20) einstückig sind.

8. Intravaskuläre Vorrichtung nach Anspruch 7, wobei die Rippen (30) Bereiche vergrößerter Wanddicke in der Außenwand aufweisen.

9. Intravaskuläre Vorrichtung nach Anspruch 8, wobei sich die Rippen (30) von der Außenwand (20) nach außen erstrecken.

10. Intravaskuläre Vorrichtung nach Anspruch 8, wobei sich die Rippen (30) nach innen erstrecken.

11. Intravaskuläre Vorrichtung nach Anspruch 1, wobei der eine oder die mehreren Bereiche modifizierter Flexibilität eine Kombination aus einem oder mehreren Bereichen verringerter Wanddicke in der Außenwand und einem oder mehreren Rippen, die sich in Längsrichtung entlang der Außenwand erstrecken, aufweisen.

12. Intravaskuläre Vorrichtung nach Anspruch 11, wobei der eine oder die mehreren Rippen (30) in bezug auf die Längsmittelachse gegenüberliegend zur Innenwand (24) positioniert sind.

13. Intravaskuläre Vorrichtung nach Anspruch 12, wobei der eine oder die mehreren Rippen (30) äquidistant von der Innenwand positioniert sind.

14. Intravaskuläre Vorrichtung nach Anspruch 12, wobei der eine oder die mehreren Rippen (30) gleichmäßig beabstandet sind.

## Revendications

1. Dispositif intravasculaire comprenant une gaine tubulaire allongée (12) possédant une paroi externe (20) et une paroi interne (24) qui subdivise la paroi externe (20) en une première et une deuxième lumières longitudinales, et une ou plusieurs zones de flexibilité modifiée s'étendant en direction longitudinale le long de la paroi externe pour réduire un déséquilibre de matière et de flexibilité autour de l'axe central longitudinal de la gaine (12) qui pourrait survenir en l'absence des zones de flexibilité modifiée.

2. Dispositif intravasculaire selon la revendication 1, dans lequel la première lumière est plus grande que la deuxième lumière.

3. Dispositif intravasculaire selon la revendication 2, dans lequel dans lequel la première lumière est en forme de croissant.

4. Dispositif intravasculaire selon la revendication 3, dans lequel la deuxième lumière est circulaire.

5. Dispositif intravasculaire selon la revendication 1, dans lequel lesdites une ou plusieurs zones de flexibilité modifiée comprennent une ou plusieurs zones possédant une épaisseur de paroi réduite, dans la paroi externe.

6. Dispositif intravasculaire selon la revendication 1, dans lequel lesdites une ou plusieurs zones de flexibilité modifiée comprennent une ou plusieurs épines (30) s'étendant en direction longitudinale le long de la paroi externe.

7. Dispositif intravasculaire selon la revendication 6, dans lequel les épines (30) sont solidaires de la paroi externe (20).

8. Dispositif intravasculaire selon la revendication 7, dans lequel les épines (30) comprennent des zones d'épaisseur de paroi supérieure, dans la paroi externe.

9. Dispositif intravasculaire selon la revendication 8, dans lequel les épines (30) s'étendent à l'extérieur de la paroi externe (20).

10. Dispositif intravasculaire selon la revendication 8, dans lequel les épines (30) s'étendent vers l'intérieur.

11. Dispositif intravasculaire selon la revendication 1, dans lequel lesdites une ou plusieurs zones de flexibilité modifiée comprennent une combinaison d'une ou plusieurs zones d'épaisseur de paroi réduite dans la paroi externe et d'une ou plusieurs épines s'étendant en direction longitudinale le long de la paroi externe.

12. Dispositif intravasculaire selon la revendication 11, dans lequel lesdites une ou plusieurs épines (30) sont disposées, par rapport à l'axe longitudinal central, à l'opposé de la paroi interne (24).

13. Dispositif intravasculaire selon la revendication 12, dans lequel lesdites une ou plusieurs épines (30) sont disposées en position équidistante par rapport à la paroi interne.

14. Dispositif intravasculaire selon la revendication 12, dans lequel lesdites une ou plusieurs épines (30) manifestent un écartement réciproque uniforme.
